# EUROPEAN PATENT SPECIFICATION

(11) **EP 3 716 016 B1**
(45) Date of publication and mention of the grant of the patent: **21.04.2021**
(21) Application number: 19165786.5
(22) Date of filing: 28.03.2019
(51) Int. Cl.: G06F 3/01, A61B 5/0476, A61B 5/0484, A61B 5/00

(54) **METHOD AND BRAIN-COMPUTER INTERFACE SYSTEM FOR RECOGNIZING BRAINWAVE SIGNALS**
VERFAHREN UND GEHIRN-COMPUTER-SCHNITTSTELLENSYSTEM ZUM ERKENNEN VON GEHIRNWELLENSIGNALEN
SYSTEM ET PROCÉDÉ D'INTERFACE CERVEAU-ORDINATEUR POUR RECONNAÎTRE DES SIGNAUX D'ONDES CÉRÉBRALES

(43) Date of publication of application: 30.09.2020
(73) Proprietor: National Chung-Shan Institute of Science and Technology, 325 Taoyuan City (TW)
(72) Inventor: Lee, Po-Lei, 103 Taipei City (TW); Hsu, Hao-Teng, 600 Chiayi City (TW); Tang, Shyh-Jian, 325 Taoyuan City (TW); Yeh, Chia-Lung, 324 Taoyuan City (TW); Chen, Yun-Ru, 325 Taoyuan City (TW); Chiang, Yi-Ju, 407 Taichung City (TW)
(74) Representative: Straus, Alexander

(56) References cited:
- WO-A1-2012/013535
- US-A1- 2017 035 317
- US-A1- 2018 039 329
- US-B2- 8 155 736
- PO-LEI LEE ET AL: "An SSVEP-Actuated Brain Computer Interface Using Phase-Tagged Flickering Sequences: A Cursor System", ANNALS OF BIOMEDICAL ENGINEERING, KLUWER ACADEMIC PUBLISHERS-PLENUM PUBLISHERS, NE, vol. 38, no. 7, 23 February 2010 (2010-02-23), pages 2383-2397, XP019811699, ISSN: 1573-9686

## Description

### Field of the Invention

The present disclosure relates to a method and system for processing brainwave signals acquired at brain-computer interface.

### Background of the Invention

With the development of biomedical technology, facilities for processing physiological signals are widely developed and utilized, e.g. a brain-computer interface (BCI) device utilized for recognizing electroencephalography (EEG) for recording and analysis. In a conventional technology of the BCI utilized for recognizing the electroencephalography, head-mounted devices (HMD) are involved to incorporate with the BCI interface. More specifically, a user may be equipped with the HMD to stare or gaze at a virtual image displayed on a display of the HMD, so as to activate corresponding BCI commands. Practically, a flashing object may be placed on lateral sides of the HMD display. When the user stares or gazes at the flashing object on one of the lateral sides, visual potentials of the electroencephalography are generated accordingly, such that the BCI consequently generates a corresponding command to the visual potentials.

However, when the user stares or gazes at the flashing object on one of the lateral sides of the HMD display to make a command, an attention of the user is distracted from the lateral of the HMD display, such that the user needs to stare back and forth between the HMD display and the lateral sides of the HMD display, which is incapable of reacting to a variation of the HMD display simultaneously. Moreover, a frequent change of gaze attention increases tiredness of the user. Therefore, an improvement is necessary.

The following references represent prior art relevant to the present disclosure:
U.S. application No. US 2017/035317 A1 provides a portable brain activity sensing platform for assessment of visual field deficits, which monitors electrical signals of the brain exhibited by an user and produces an assessment of the user's visual field. U.S. application No. US 2018/039329 A1 provides a brain actuated control utilizing visually evoked potentials, which attaches high impedance dry Ag/Ag-CI electrodes on a human user's scalp and analyzes the detected steady-state evoked potentials in response to visual stimuli. World Intellectual Property Organization application No. WO 2012/013535 A1 provides a brain-computer interfaces and use thereof, which decodes visual evoked potentials to determine a most likely target of interest or absence thereof. U.S. application No. US 8155736 B2 provides an EEG control of devices using sensory evoked potentials, which determines whether the received EEG signal samples are evoked in response to a pattern of stimulus. "An SSVEP-actuated brain computer interface using phase-tagged flickering sequences: a cursor system" of Annals of biomedical engineering provides a reliable channel for severely disabled patients to communicate with external environments.

### Summary of the Invention

The present invention is defined by the independent claims. The dependent claims define further advantageous embodiments.

The present disclosure relates to simultaneous processing of brainwave signals as acquired by a brain-computer interface (BCI) so as to avoid the distraction of the attention of user when activating the commands corresponding to the BCI and improve the user scenario.

### Brief Description of the Drawings

FIG. 1 is a schematic diagram of a brain-computer interface (BCI) system according to an embodiment of the present disclosure.
FIG. 2 is a schematic diagram of an Epoch-average process.
FIG. 3 is a schematic diagram of stimulus sources and a virtual image on a HMD display according to an embodiment of the present disclosure.
FIG. 4 is a schematic diagram of a method according to an embodiment of the present disclosure.

### Detailed Description

When a user stares at an object, sights perceived by eyes would be transferred to the visual cortex of the brain through the lateral optic nerves and the lateral geniculate nucleus of thalamus. In a brain-computer interface (BCI) system, brainwave-signal electrodes may be respectively placed on positions of an O1 electrode channel, 02 electrode channel and Oz electrode channel on a head of a user, so as to measure evoked potential signals corresponding to the Oz electrode channel, the O1 electrode channel and the 02 electrode channel based on the International 10-20 system.

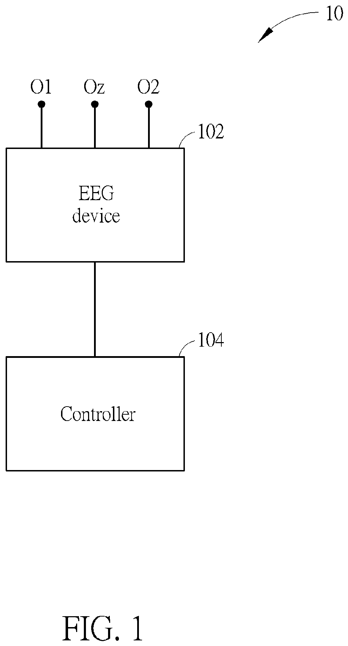

Please refer to FIG. 1, which is a schematic diagram of a brain-computer interface (BCI) system 10 according to an embodiment of the present disclosure. The BCI system 10 includes an electroencephalography (EEG) device 102 and a controller 104. The EEG device 102 is configured to acquire brainwave signals from a user. In an embodiment, the EEG device 102 may record or monitor electrical activities of the brainwave signals of the user. The controller 104 is coupled to the EEG device 102 and configured to measure a steady-state visual evoked potential (SSVEP) corresponding to an Oz electrode channel according to the brainwave signals when in a rest state, measure the SSVEPs respectively corresponding to the Oz electrode channel, an O1 electrode channel and an 02 electrode channel according to the brainwave signals when in a stimulating state, and determine a stimulus source of a gaze attention according to the SSVEPs of the O1 electrode channel and the SSVEP of the 02 electrode channel. That is, the EEG device 102 records the brainwave signals before and after the user is stimulated by a stimulus source, and then the controller 104 measures the SSVEPs corresponding to the O1 electrode channel and the SSVEP of the 02 electrode channel. Therefore, the BCI system 10 of the present disclosure may simultaneously respond to a glance attention made by the user and recognize the brainwave signals.

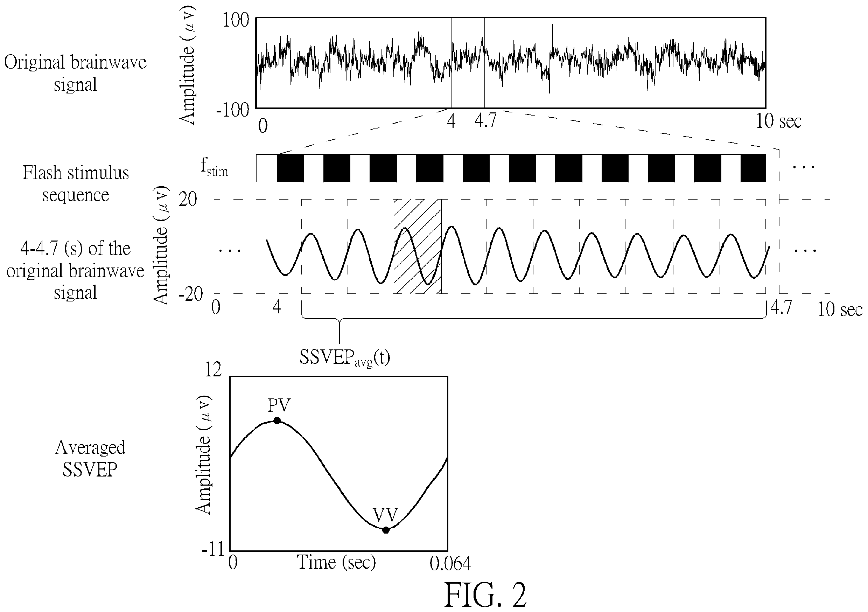

Since the steady state potentials evoked by a lateral view of the user are weaker than that evoked by a direct view, an Epoch-averaged process is applied to increase a signal to noise ratio (SNR) and extract the steady state potentials evoked by the lateral view. Please refer to FIG. 2, which is a schematic diagram of the Epoch-average process. In FIG. 2, an original brainwave signal of the Oz electrode channel for 10 seconds is obtained. A flash stimulus sequence fstim with a stimulating flash frequency at 30 Hz is generated based on the original brainwave signal between 4 and 4.7 seconds, which is performed a band-pass filtering between 20 and 31 Hz. The flash stimulus sequence fstim consists of a plurality of dark periods and a plurality of bright periods corresponding to the original brainwave signal, and an onset point is defined at a transition of a dark period and a bright period. In an embodiment, a period between two onset points is defined as an Epoch. After the latest 10 Epochs in the flash stimulus sequence fstim are determined based on the onset points for averaging, an averaged SSVEPavg(t) is determined. In the example, an amplitude of the averaged SSVEPavg(t) is between -11 microvolt(µv) and 12 µv, and the averaged SSVEPavg(t) is determined from a difference of a maximal amplitude PV and a minimal amplitude VV, which is SSVEPfstim = PV-VV (µv).

In an embodiment of the present disclosure, the brainwave signals of the Oz electrode channel, the O1 electrode channel and the 02 electrode channel are respectively recorded by the EEG 102, such that the controller 104 respectively measures the SSVEPs of the Oz electrode channel, the O1 electrode channel and the O2 electrode channel based on the Epoch-average process.

In an example, the BCI system 10 may further include a head-mounted device (HMD) to display virtual images to the user, such that the user may make commands through the glance attentions. When the user glances at a right side of a HMD display, the brainwave signals (e.g. optic nerve signals) are transferred to a left side of the visual cortex of the brain. In contrast, when the user glances at a left side of the HMD display, the brainwave signals are transferred to a right side of the visual cortex of the brain. In such a situation, the brainwave signals corresponding to different regions of the visual cortex of the brain may be taken as controlling signals of the BCI system 10. Therefore, the BCI system 10 utilizes the glance attention of the user to the stimulus source to determine the commands of the user.

In an operation process of the BCI system 10, the EEG device 102 may record the brainwave signals for a period, e.g. at least 10 seconds, before the user is stimulated or when the brain of the user is in the rest state. The brainwave signals are utilized for measuring the SSVEP of the Oz electrode channel by the controller 104 based on the Epoch-average process. Notably, the glance attention of the user triggers/activates the brainwave signals when in the stimulating state. In an example, different flash stimulus sequences at different stimulating flash frequencies may be determined or classified, which may correspond to different stimuli, e.g. different regions of the lateral HMD display respectively correspond to different stimuli glanced by the user. In addition, a threshold related to a maximum of the SSVEP of the Oz electrode channel when in the rest state is measured to determine whether the SSVEP of the 01 electrode channel is greater than the SSVEP of the 02 electrode channel when the SSVEP of the Oz electrode channel is greater than the threshold.

When the SSVEP of the O1 electrode channel is greater than the SSVEP of the O2 electrode channel in the stimulating state, the controller 104 determines that a direction of the stimulus source of the glance attention of the user is towards the right side of the HMD display, which represents a command made by the user. Similarly, when the SSVEP of the O1 electrode channel is lower than the SSVEP of the 02 electrode channel in the stimulating state, the controller 104 determines that the direction of the stimulus source of the glance attention of the user is towards the left side of the HMD display. Therefore, when the user operates the BCI system 10, the user is free from gazing at the corresponding region of the lateral HMD display to make commands. Instead, the user focuses on the HMD display and glances at the lateral region of the HMD display to make commands for the BCI system 10.

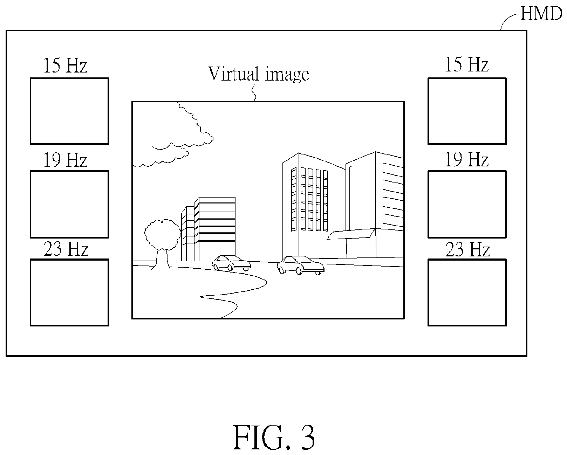

Moreover, the BCI system 10 of the present disclosure may be implemented in all kinds of ways. Since the different flash stimulus sequences at different stimulating flash frequencies correspond to different stimulus sources on the HMD display, the different regions of the lateral HMD display may correspond to different commands. Please refer to FIG. 3, which is a schematic diagram of the stimulus sources and the virtual image on the HMD display according to an embodiment of the present disclosure. As shown in FIG. 3, each of the stimulating flash frequencies corresponds to a region on the lateral HMD display glanced by the user. In a user scenario, the user with the HMD operates with the BIC system 10 and makes commands to move a wheelchair. After the controller 104 measures the SSVEPs of the Oz electrode channel, the O1 electrode channel and the 02 electrode channel before and after the stimulating state, the controller 104 measures whether the SSVEP of the O1 electrode channel is greater than the SSVEP of the 02 electrode channel to determine the stimulus source of the glance attention made by the user. The stimulating flash frequency at 15 Hz of the right side of the HMD display may correspond to a command of "turn right" in the BCI system 10, the stimulating flash frequency at 15 Hz of the left side of the HMD display may correspond to a command of "turn left" in the BCI system 10, the stimulating flash frequency at 19 Hz of the right side of the HMD display may correspond to a command of "move forward" in the BCI system 10, and the stimulating flash frequency at 19 Hz of the left side of the HMD display may correspond to a command of "move back" in the BCI system 10. Similarly, the stimulating flash frequency at 19 Hz of the right side and the left side of the HMD display respectively correspond to different commands in the BCI system 10.

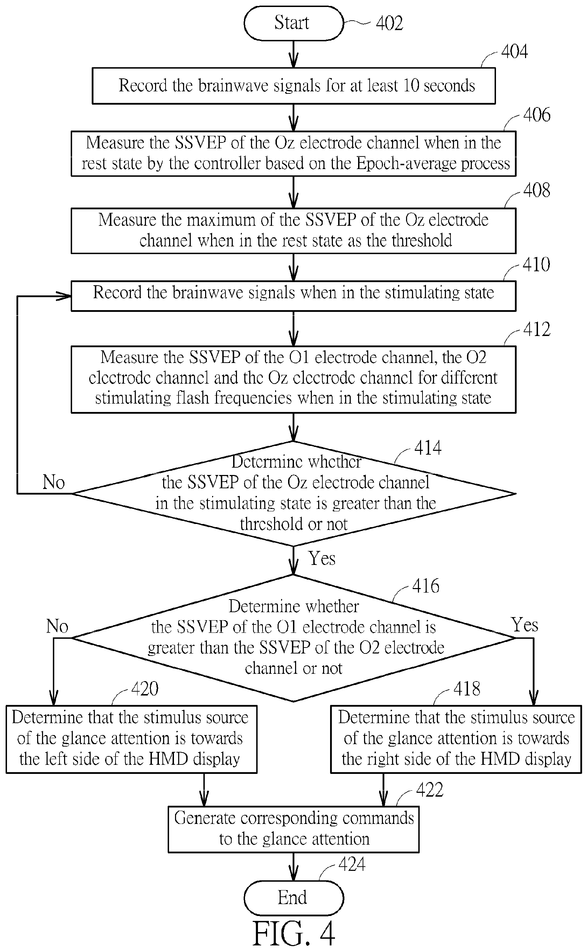

An operation process of the BCI system 10 may be summarized to a method 40 for recognizing the brainwave signals of the BCI system 10 as shown in to FIG. 4, which is a schematic diagram of a method 40 according to an embodiment of the present disclosure. The method 40 includes the following steps:
Step 402: Start.
Step 404: Record the brainwave signals for at least 10 seconds.
Step 406: Measure the SSVEP of the Oz electrode channel when in the rest state by the controller 104 based on the Epoch-average process.
Step 408: Measure the maximum of the SSVEP of the Oz electrode channel when in the rest state as the threshold.
Step 410: Record the brainwave signals when in the stimulating state.
Step 412: Measure the SSVEP of the O1 electrode channel, the 02 electrode channel and the Oz electrode channel for different stimulating flash frequencies when in the stimulating state.
Step 414: Determine whether the SSVEP of the Oz electrode channel in the stimulating state is greater than the threshold or not. If yes, execute step 416; if no, execute step 410.
Step 416: Determine whether the SSVEP of the O1 electrode channel is greater than the SSVEP of the 02 electrode channel or not. If yes, execute step 418; if no, execute step 410.
Step 418: Determine that the stimulus source of the glance attention is towards the right side of the HMD display.
Step 420: Determine that the stimulus source of the glance attention is towards the left side of the HMD display.
Step 422: Generate corresponding commands to the glance attention.
Step 424: End.

The operation of the method 40 can be known by referring to the embodiments of the BCI system 10 described above, and are not repeated herein for brevity.

Notably, the embodiments stated above illustrate the concept of the present disclosure, those skilled in the art may make proper modifications accordingly, and not limited thereto. For example, the different regions of the lateral HMD display may respectively correspond to different commands of the BCI system, flash stimulus sequences at different stimulating flash frequencies may be determined to correspond different stimulus sources on the HMD display, and not limited thereto.

In summary, the present disclosure provides a method and system for processing the brainwave signals of the BCI system, which avoids the distraction of the attention of the user when activating the commands corresponding to the BCI interface. Instead, the present disclosure recognizes the brainwave signals corresponding to the glance attention of the user to generate corresponding commands in the BCI system and improve the user scenario.

## Claims

1. A computer-implemented method (40) for processing brainwave signals of a user as acquired by a brain-computer interface (10), abbreviated to BCI, system, said BCI system(10) comprising an electroencephalography, abbreviated to EEG, device (102), said method comprising the steps of: in a rest state:
measuring via the EEG device a steady-state visual evoked potential, abbreviated to SSVEP, corresponding to an Oz electrode channel according to the brainwave signals (404);
in a stimulating state, while a plurality of visual stimuli are displayed at a display:
measuring via the EEG device the SSVEP of the Oz electrode channel according to the brainwave signals (410) and determining (414) whether it is greater than a threshold based on the rest state SSVEP measurement;
measuring via the EEG device the SSVEPs respectively corresponding to an O1 electrode channel and an 02 electrode channe (410) according to the brainwave signals when in the stimulating state (412); and
determining a direction of a glance attention of the user according to the SSVEPs of the O1 electrode channel and the SSVEP of the 02 electrode channel (414, 416);
wherein the determination of the glance attention of the user is used to determine and activate a command corresponding to one of the plurality of visual stimuli (422);
wherein the step of determining the direction of the glance attention according to the SSVEPs of the O1 electrode channel and the SSVEP of the 02 electrode channel (414, 416) comprises: when the SSVEP of the Oz electrode channel is greater than the threshold (414),
determining whether the SSVEP of the O1 electrode channel is greater than the SSVEP of the 02 electrode channel to determine the direction of the glance attention of the stimulus source.

2. The method (40) of claim 1, wherein the threshold is related to a maximum of the SSVEP of the Oz electrode channel when in the rest state.

3. The method (40) of claim 1, further comprising, when the SSVEP of the O1 electrode channel is greater than the SSVEP of the 02 electrode channel when in the stimulating state, determining that the stimulus source of the glance attention is towards a right side (418).

4. The method (40) of claim 1, further comprising, when the SSVEP of the O1 electrode channel is lower than the SSVEP of the 02 electrode channel when in the stimulating state, determining that the stimulus source of the glance attention is towards a left side (420).

5. The method (40) of claim 1, wherein the SSVEPs of the Oz electrode channel, the O1 electrode channel and the O2 electrode channel are determined based on an epoch-averaged process (406).

6. A brain-computer interface, abbreviated to BCI, system (10), comprising:
an electroencephalography, abbreviated to EEG, device (102), configured to acquire brainwave signals of a user; and
a controller (104), coupled to the EEG device (102) and configured to measure a steady-state visual evoked potential, abbreviated to SSVEP, corresponding to an Oz electrode channel according to the brainwave signals in a rest state(404);
trigger a stimulating state by displaying a plurality of visual stimuli at a display and, in said stimulating state:
measure
the SSVEP of the Oz electrode channel (410)
according to the brainwave signals and determine (414) whether it is greater than a threshold based on the rest state SSVEP measurement, measure the SSVEPs respectively corresponding to an 01 electrode channel and an 02 electrode channel according to the brainwave signals when in the stimulating state (412), and
determine a direction of a glance attention of the user according to the SSVEPs of the O1 electrode channel and the SSVEP of the 02 electrode channel (414, 416);
wherein the determination of the glance attention of the user is used to determine and activate a command corresponding to one of the plurality of visual stimuli (422);
wherein, when the SSVEP of the Oz electrode channel is greater than the threshold (414), the controller (104) determines whether the SSVEP of the O1 electrode channel is greater than the SSVEP of the 02 electrode channel to determine the direction of the glance attention of the stimulus source.

7. The BCI system (10) of claim 6, wherein the threshold is related to a maximum of the SSVEP of the Oz electrode channel when in the rest state.

8. The BCI system (10) of claim 6, wherein the controller (104) determines that the stimulus source of the glance attention is towards a right side when the SSVEP of the O1 electrode channel is greater than the SSVEP of the 02 electrode channel when in the stimulating state (418).

9. The BCI system (10) of claim 6, wherein the controller (104) determines the stimulus source of the glance attention is towards a left side when the SSVEP of the O1 electrode channel is lower than the SSVEP of the 02 electrode channel when in the stimulating state (420).

10. The BCI system (10) of claim 6, wherein the SSVEP is determined based on an Epoch-averaged process (406).

## Patentansprüche

1. Computerimplementiertes Verfahren (40) zum Prozessieren von Hirnwellensignalen eines Nutzers, die durch ein Hirn-Computer-Interface (10), abgekürzt BCI-System, erfasst wurden, worin das BCI-System (10) ein Elektroenzephalographie-, abgekürzt EEG, Gerät (102) umfasst, wobei das Verfahren die Schritte umfasst:
in einem Ruhezustand:
Erfassen eines visuell-evozierten Stabilzustandspotenziales, abgekürzt SSVEP, mittels des EEG-Gerätes entsprechend einem Oz-Elektrodenkanal basierend auf den Hirnwellensignalen (404);
in einem Anregungszustand, während mehrere visuelle Stimuli in einer Anzeige angezeigt werden:
Erfassen des SSVEP des Oz-Elektrodenkanals basierend auf den Hirnwellensignalen (410) mittels des EEG-Gerätes und
Bestimmen (414), ob dieses größer ist als ein Schwellenwert bezogen auf die SSVEP-Ruhezustandserfassung;
Erfassen der entsprechenden SSVEPs entsprechend einem O1-Elektrodenkanal und einem O2-Elektrodenkanal (410) basierend auf den Hirnwellensignalen mittels des EEG-Gerätes, wenn der Anregungszustand (412) vorliegt; und
Bestimmen einer Richtung einer Blickaufmerksamkeit des Nutzers entsprechend dem SSVEP des O1-Elektrodenkanals und dem SSVEP des O2-Elektrodenkanals (414, 416);
wobei die Bestimmung der Blickaufmerksamkeit des Nutzers verwendet wird, um entsprechend einem den mehreren visuellen Stimuli (422) einen Befehl zu bestimmen und zu aktivieren;
wobei der Schritt des Bestimmens der Richtung der Blickaufmerksamkeit entsprechend dem SSVEP des O1-Elektrodenkanals und dem SSVEP des O2-Elektrodenkanals (414, 416) umfasst:
wenn das SSVEP des Oz-Elektrodenkanals größer ist als der Schwellenwert (414), Bestimmen, ob das SSVEP des O1-Elektrodenkanals größer ist als das SSVEP des O2-Elektrodenkanals, um die Richtung der Blickaufmerksamkeit auf die Stimulusquelle zu bestimmen.

2. Verfahren (40) nach Anspruch 1, wobei der Schwellenwert auf ein Maximum des SSVEP des Oz-Elektrodenkanals bezogen ist, wenn der Ruhezustand vorliegt.

3. Verfahren (40) nach Anspruch 1, welches weiter umfasst, Bestimmen, dass die Stimulusquelle der Blickaufmerksamkeit einer rechten Seite zugewandt ist (418), wenn das SSVEP des O1-Elektrodenkanals größer ist als das SSVEP des O2-Elektrodenkanals, wenn der Anregungszustand vorliegt.

4. Verfahren (40) nach Anspruch 1, welches weiter umfasst, Bestimmen, dass die Stimulusquelle der Blickaufmerksamkeit einer linken Seite zugewandt ist (420), wenn das SSVEP des O1-Elektrodenkanals geringer ist als das SSVEP des O2-Elektrodenkanals, wenn der Anregungszustand vorliegt.

5. Verfahren (40) nach Anspruch 1, wobei die SSVEPs des Oz-Elektrodenkanals, des O1-Elektrodenkanals und des O2-Elektrodenkanals bezogen auf einen Epochen-gemittelten Prozess (406) bestimmt werden.

6. Hirn-Computer-Interface-, abgekürzt BCI, System (10), welches umfasst:
ein Elektroenzephalographie-, abgekürzt EEG, Gerät (102), das ausgestaltet ist, Hirnwellensignale eines Nutzers zu erfassen; und
eine Steuerung (104), die mit dem EEG-Gerät (102) gekoppelt und ausgestaltet ist, ein visuell evoziertes Stabilzustandspotenzial, abgekürzt SSVEP, entsprechend einem Oz-Elektrodenkanal basierend auf den Hirnwellensignalen in einem Ruhezustand (404) zu erfassen;
Auslösen eines Anregungszustandes durch Anzeigen mehrerer visueller Stimuli auf einer Anzeige und, wenn der Anregungszustand vorliegt:
Erfassen des SSVEP des Oz-Elektrodenkanals (410) basierend auf den Hirnwellensignalen und Bestimmen (414), ob dieses größer ist als ein Schwellenwert bezogen auf die SSVEP-Ruhezustandserfassung,
Erfassen der SSVEPs entsprechend einem O1-Elektrodenkanal und einem O2-Elektrodenkanal basierend auf den Hirnwellensignalen, wenn der Anregungszustand (412) vorliegt, und
Bestimmen einer Richtung einer Blickaufmerksamkeit des Nutzers bezogen auf das SSVEP des O1-Elektrodenkanals und das SSVEP des O2-Elektrodenkanals (414, 416);
wobei die Bestimmung der Blickaufmerksamkeit des Nutzers verwendet wird, um entsprechend einem der mehreren visuellen Stimuli (422) einen Befehl zu bestimmen und zu aktivieren;
wobei wenn das SSVEP des Oz-Elektrodenkanals größer ist als ein Schwellenwert (414), die Steuerung (104) bestimmt, ob das SSVEP des O1-Elektrodenkanals größer ist als das SSVEP des O2-Elektrodenkanals, um die Richtung der Blickaufmerksamkeit auf die Stimulusquelle zu bestimmen.

7. BCI-System (10) nach Anspruch 6, worin der Schwellenwert auf ein Maximum des SSVEP des Oz-Elektrodenkanals bezogen ist, wenn der Ruhezustand vorliegt.

8. BCI-System (10) nach Anspruch 6, worin die Steuerung (104) bestimmt, dass die Stimulusquelle der Blickaufmerksamkeit einer rechten Seite zugewandt ist, wenn das SSVEP des O1-Elektrodenkanals größer ist als das SSVEP des O2-Elektrodenkanals, wenn der Anregungszustand (418) vorliegt.

9. BCI-System (10) nach Anspruch 6, worin die Steuerung (104) bestimmt, dass die Stimulusquelle der Blickaufmerksamkeit einer linken Seite zugewandt ist, wenn das SSVEP des O1-Elektrodenkanals geringer ist als das SSVEP des O2-Elektrodenkanals, wenn der Anregungszustand (420) vorliegt.

10. BCI-System (10) nach Anspruch 6, worin das SSVEP bezogen auf einen Epochen-gemittelten Prozess (406) bestimmt wird.

## Revendications

1. Procédé mis en œuvre par ordinateur (40) pour traiter des signaux d'ondes cérébrales d'un utilisateur tels qu'acquis par un système d'interface cerveau-ordinateur (10), en abrégé BCI, ledit système BCI (10) comprenant un dispositif d'électroencéphalographie, en abrégé EEG, (102), ledit procédé comprenant les étapes de :
dans un état de repos :
mesure, par l'intermédiaire du dispositif EEG, d'un potentiel évoqué visuel à l'état stable, en abrégé SSVEP, correspondant à un canal d'électrode Oz en fonction des signaux d'ondes cérébrales (404);
dans un état de stimulation, tandis qu'une pluralité de stimuli visuels sont affichés au niveau d'un dispositif d'affichage :
mesure, par l'intermédiaire du dispositif EEG, du SSVEP du canal d'électrode Oz en fonction des signaux d'ondes cérébrales (410) et détermination (414) du fait qu'il est supérieur ou non à un seuil sur la base de la mesure SSVEP à l'état de repos;
mesure, par l'intermédiaire du dispositif EEG, des SSVEP correspondant respectivement à un canal d'électrode O1 et à un canal d'électrode O2 (410) en fonction des signaux d'ondes cérébrales dans l'état de stimulation (412) ; et
détermination d'une direction d'une attention du regard de l'utilisateur en fonction des SSVEP du canal d'électrode O1 et du SSVEP du canal d'électrode O2 (414, 416) ;
la détermination de l'attention du regard de l'utilisateur étant utilisée pour déterminer et activer une commande correspondant à un stimulus parmi la pluralité de stimuli visuels (422) ;
l'étape de détermination de la direction de l'attention du regard en fonction des SSVEP du canal d'électrode O1 et du SSVEP du canal d'électrode O2 (414, 416) comprenant :
lorsque le SSVEP du canal d'électrode Oz est supérieur au seuil (414), la détermination du fait que le SSVEP du canal d'électrode O1 est supérieur ou non au SSVEP du canal d'électrode O2 pour déterminer la direction de l'attention du regard de la source de stimulus.

2. Procédé (40) selon la revendication 1, le seuil étant lié à un maximum du SSVEP du canal d'électrode Oz dans l'état de repos.

3. Procédé (40) selon la revendication 1, comprenant en outre, lorsque le SSVEP du canal d'électrode O1 est supérieur au SSVEP du canal d'électrode O2 dans l'état de stimulation, la détermination du fait que la source de stimulus de l'attention du regard est vers un côté droit (418).

4. Procédé (40) selon la revendication 1, comprenant en outre, lorsque le SSVEP du canal d'électrode O1 est inférieur au SSVEP du canal d'électrode O2 dans l'état de stimulation, la détermination du fait que la source de stimulus de l'attention du regard est vers un côté gauche (420).

5. Procédé (40) selon la revendication 1, les SSVEP du canal d'électrode Oz, du canal d'électrode O1 et du canal d'électrode O2 étant déterminés sur la base d'un processus moyenné par fenêtre temporelle (406).

6. Système (10) d'interface cerveau-ordinateur, en abrégé BCI (10), comprenant :
un dispositif d'électroencéphalographie, en abrégé EEG, (102), configuré pour acquérir des signaux d'ondes cérébrales d'un utilisateur ; et
un dispositif de commande (104), couplé au dispositif EEG (102) et configuré pour mesurer un potentiel évoqué visuel à l'état stable, en abrégé SSVEP, correspondant à un canal d'électrode Oz en fonction des signaux d'ondes cérébrales dans un état de repos (404) ; déclencher un état de stimulation par l'affichage d'une pluralité de stimuli visuels au niveau d'un dispositif d'affichage et, dans ledit état de stimulation :
mesurer le SSVEP du canal d'électrode Oz (410) en fonction des signaux d'ondes cérébrales et déterminer (414) s'il est supérieur ou non à un seuil sur la base de la mesure SSVEP à l'état de repos,
mesurer les SSVEP correspondant respectivement à un canal d'électrode O1 et à un canal d'électrode O2 en fonction des signaux d'ondes cérébrales dans l'état de stimulation (412), et
déterminer une direction d'une attention du regard de l'utilisateur en fonction des SSVEP du canal d'électrode O1 et du SSVEP du canal d'électrode O2 (414, 416) ;
la détermination de l'attention du regard de l'utilisateur étant utilisée pour déterminer et activer une commande correspondant à un stimulus parmi la pluralité de stimuli visuels (422) ;
où, lorsque le SSVEP du canal d'électrode Oz est supérieur au seuil (414), le dispositif de commande (104) détermine si le SSVEP du canal d'électrode O1 est supérieur ou non au SSVEP du canal d'électrode O2 pour déterminer la direction de l'attention du regard de la source de stimulus.

7. Système BCI (10) selon la revendication 6, le seuil étant lié à un maximum du SSVEP du canal d'électrode Oz dans l'état de repos.

8. Système BCI (10) selon la revendication 6, le dispositif de commande (104) déterminant que la source de stimulus de l'attention du regard est vers un côté droit lorsque le SSVEP du canal d'électrode O1 est supérieur au SSVEP du canal d'électrode O2 dans l'état de stimulation (418).

9. Système BCI (10) selon la revendication 6, le dispositif de commande (104) déterminant que la source de stimulus de l'attention du regard est vers un côté gauche lorsque le SSVEP du canal d'électrode O1 est inférieur au SSVEP du canal d'électrode O2 dans l'état de stimulation (420).

10. Système BCI (10) selon la revendication 6, le SSVEP étant déterminé sur la base d'un processus moyenné par fenêtre temporelle (406).
